# EUROPEAN PATENT SPECIFICATION

(11) **EP 3 751 006 B1**
(45) Date of publication and mention of the grant of the patent: **24.04.2024**
(21) Application number: 20179784.2
(22) Date of filing: 12.06.2020
(51) Int. Cl.: C12Q 1/686, C12Q 1/70

(54) **EXTERNAL CONTROL FOR NUCLEIC ACID AMPLIFICATION**
EXTERNE CONTROLLE FÜR NUKLEINSÄUREAMPLIFIKATION
CONTRÔLE EXTERNE POUR L'AMPLIFICATION D'ACIDES NUCLÉIQUES

(30) Priority: 12.06.2019 IT 201900008781
(43) Date of publication of application: 16.12.2020
(73) Proprietor: AB Analitica S.r.l., 35127 Padova (PD) (IT)
(72) Inventor: BALDANTI, Fausto, 29011 Borgonovo Val Tidone (IT); GANI, Anna, 35122 Padova (IT); COSTACURTA, Riccardo, 35010 Vigonza (IT); PACCAGNELLA, Nicola, 35030 Bosco di Rubano (IT); ZANCAN, Irene, 35020 Terradura di Due Carrare (IT); PALADIN, Dino, 35129 Padova (IT)
(74) Representative: Ghirardi, Valeria

(56) References cited:
- EP-A1- 3 208 333
- WO-A2-02/16648
- GB-A- 2 525 024
- MUNRO J ET AL: "Development of a Synthetic Positive Control Which Also Detects Plasmid Contamination in Diagnostic Polymerase Chain Reaction", MOLECULAR BIOLOGY, KLUWER ACADEMIC PUBLISHERS-PLENUM PUBLISHERS, NE, vol. 39, no. 6, 1 November 2005 (2005-11-01), pages 915-917, XP019288407, ISSN: 1608-3245
- M A Lee ET AL: "Internal and External Controls for Reagent Validation", , 1 January 2008 (2008-01-01), XP055671632, Retrieved from the Internet: URL:https://www.gene-quantification.de/lee -et-al-horizonpress-2008.pdf [retrieved on 2020-02-25]

## Description

### FIELD OF THE INVENTION

The present invention relates to an *in vitro* or *ex vivo* method for carrying out an amplification of a target nucleotide sequence by a polymerase chain reaction (PCR), a nucleic acid molecule to be used as a single control in the PCR reaction, and a nucleic acid amplification kit.

### PRIOR ART

In every qualitative or quantitative PCR (polymerase chain reaction) experimental session dedicated to diagnostics, reaction controls are usually inserted, i.e., a positive and a negative control within two separate reaction tubes. The negative control ("No Template Control",NTC) consists of the reaction mixture without the addition of nucleic acid template, instead of which a so-called "white reaction" consisting of water or buffer is added. The negative control is used to detect any contamination which may have been introduced in the PCR set-up step and which could lead to false positive results for the test samples. The positive control PC, instead consists of a "sample" in which the target sequence is definitely present and its purpose is to act as an indicator of the possible failure of the PCR, due to the malfunction of one of the reagents or of the instrument (Clinical and Laboratory Standards Institute (CLSI). Molecular Diagnostic Methods for Infectious Disease. 3rd. CLSI report MM03). In this way at least two reaction tubes are assigned to the execution of the reaction controls, for each assay being examined. As a result, using two controls is a disadvantage from several points of view. For example, this brings a higher analysis cost because more reagents are used. In addition, fewer samples will be processable in a multi-target session because two wells are used for the controls for each target. In addition, a further disadvantage is the possible increase in error due to the fact that the controls may not be subject to the same conditions.

PCR kits and positive controls are described in several patent applications. For example, application US2018320218 relates to a PCR kit, comprising a vessel and at least two sets of reagents arranged separately in the vessel, in which each of the at least two groups of reagents comprises at least one component selected from the group consisting of a DNA polymerase, nucleoside triphosphates, reaction buffer and salt and provided that each set of at least two sets of reagents is different from one another and that the combination of each of the at least two sets of reagents comprises a DNA polymerase, nucleoside triphosphates, reaction buffer and salt necessary to perform the PCR.

International Application WO0233129 relates to a positive control material for identification based on nucleic acid amplification of microorganisms in biological samples. The control material comprises a purified microorganism rendered non-infectious but susceptible to nucleic acid amplification. A process for creating and using the control material is also described. International application WO2013148212 relates to nucleic acid sequences which can be used for nucleic acid amplification EP3208333A1 describes the use of a housekeeping gene and of an exogenous nucleic acid as internal positive controls, each having different amplification sequences.

There is therefore still a need to develop a method for amplifying nucleic acids which overcomes the above-mentioned disadvantages of using two different controls.

### SUMMARY OF THE INVENTION

The aim of the present invention is to combine the reactions involving positive control (PC) and negative control (NTC) in a qualitative or quantitative PCR assay, in particular for diagnostic purposes, by inserting a single control reaction encompassing both. In particular, the "Pan Control" reaction object of the present invention allows to ascertain the total absence of contamination, a requirement normally met by the absence of signal in the NTC, and the correct functioning of the reaction reagents, usually monitored with the addition of the positive control (PC). The insertion of the "Pan Control" therefore allows only one reaction tube to be used for the validation of an analytical assay.

### DETAILED DESCRIPTION OF THE INVENTION

Therefore an object of the invention is an *ex vivo* or *in vitro* method for carrying out an amplification of at least one target nucleotide sequence by a polymerase chain reaction (PCR), comprising the following steps:
a) providing a first environment comprising at least one sample possibly containing said target nucleotide sequence;
b) providing a second environment comprising a single control to ascertain the absence of contamination and the functioning of the reaction reagents,
   said single control containing a nucleotide sequence comprising:
   - an amplifiable nucleic acid having a different or partially different sequence from said target nucleotide sequence and
   - two hybridization sites for two primers, respectively at the 5' and 3' ends of said amplifiable nucleic acid, and
   - a region inside the primer hybridization sites for the hybridization of a probe having a different sequence from said target nucleotide sequence,
c) adding a mixture to said sample and to said single control comprising:
   - two primers capable of triggering the PCR reaction of the target nucleotide sequence;
   - two primers capable of triggering the PCR reaction of the single control;
   - a first specific probe for the target nucleotide sequence;
   - a second specific probe for the amplifiable sequence of the single control;
   - appropriate reagents;
d) performing the PCR reaction on said mixture to obtain and detect copies of the target nucleotide sequence if present in said sample and copies of the nucleotide sequence of the single control, wherein the two primers capable of triggering the PCR reaction of the single control are also capable of triggering the PCR reaction of the nucleotide sequence of an exogenous target previously added to the sample of step a and wherein preferably in step c) the mixture further comprises a third specific probe for the nucleotide sequence of said exogenous target.

In the method according to the invention, the target nucleotide sequence may be one or more than one. For example, a triplex or quadruplex type reaction may be performed. The sample of step a) may also be one or more than one.

In a preferred embodiment, the probes are marked with fluorophores different from one another. Preferably, said single control consists of a vector or a linear DNA strand, preferably of at least 50 nucleotides.

Preferably, the amplifiable nucleic acid contained in the control has at least one of the following characteristics:
- GC content of around 30-80%;
- Sequence of homopolymers with a length less than 30 bp;
- Repeated sequence with a length less than 20 bp for G/C;
- Repeated sequence with a length less than 40 bp for A/T;
- Does not contain secondary hairpin type structure.

In a preferred embodiment, the method according to the invention comprises, before step c), step b') of providing a third environment comprising a sample containing a nucleotide sequence of an endogenous gene, preferably a housekeeping gene, or exogenous target, and where in step c) the mixture further comprises:
- two primers capable of triggering the PCR reaction of the nucleotide sequence of said housekeeping gene;
- a third specific probe for the nucleotide sequence of said endogenous gene or exogenous target said mixture being added also to said sample containing the nucleotide sequence of said housekeeping gene.

The third environment may correspond to the first environment above, the endogenous gene already being present in the sample possibly containing said target nucleotide sequence or the exogenous target being added to said sample previously.

Preferably, the two primers capable of triggering the PCR reaction of the control correspond to the two primers capable of triggering the PCR reaction of the nucleotide sequence of said endogenous gene or exogenous target.

In a preferred aspect, the single control comprises or consists of a sequence having an identity of at least 80% with the sequence of SEQ ID NO: 1.

In a preferred aspect, the single control comprises or consists of a sequence having an identity of at least 80% with the sequence which goes from nt. 4 to nt. 160 of SEQ ID NO: 1.

Preferably, said mixture of step c) comprises a forward primer (direct) comprising a sequence having an identity of at least 80% with the sequence CCTGTCTTGTAACCTTGATACCAA (SEQ ID NO: 5), a reverse primer comprising a sequence having an identity of at least 80% with the sequence ATGGTGCAGCTGACTCCTGA (SEQ ID NO:6) and a probe comprising a sequence having an identity of at least 80% with the sequence ACGCCTTGATTGACAAGGATGGATGGC (SEQ ID NO:8).

Preferably, the method according to the invention is a diagnostic method, for example for the detection of one or more cancer-related genes, oncogenes, viral genes, bacterial genes, fungal genes or genes involved in pathologies selected from autoimmune diseases, inflammatory diseases, infectious diseases, metabolic disorders, degenerative diseases, neurological diseases, heart, bone, joint, muscle, brain, reproductive organs, blood, lymphatic tissues, glandular tissues, breast, digestive tracts, respiratory tract or skin diseases.

Preferably the PCR is a qualitative PCR, absolute quantitative PCR, allelic discrimination, multiplex PCR, Reverse transcription PCR, High Resolution Melting (HRM), Melting analysis, Relative quantification, preferably an RT-PCR, a real-time PCR, a Droplet Digital PCR or a quantitative real-time PCR-RT.

Within the context of the present invention, the PCR is preferably a real-time PCR or Droplet Digital PCR.

A further object of the invention is the use of a nucleic acid molecule as a single control in a polymerase chain reaction (PCR), as defined above.

Another object of the invention is the use of a nucleic acid molecule as a single control in a polymerase chain reaction (PCR), said single control comprising a nucleotide sequence comprising:
- an amplifiable nucleic acid having a different or partially different sequence from said target nucleotide sequence and
- two hybridization sites for two primers, respectively at the 5 'and 3' ends of said amplifiable nucleic acid, and
- a region inside the primer hybridization sites for the hybridization of a probe having a different sequence from said target nucleotide sequence.

Preferably, in said molecule the hybridization sites for primers comprise a sequence with a homology percentage of at least 90% with respect to the sequence of an endogenous gene or an exogenous target to be used as control and/or with respect to the target nucleotide sequence.

Preferably, in the molecule according to the invention the two primer hybridization sites are capable of hybridizing primers capable of triggering the PCR reaction of the nucleotide sequence of an endogenous gene or of exogenous target and/or of target nucleotide sequence. More preferably, said two primer hybridization sites are capable of hybridizing primers capable of triggering both the PCR reaction of the nucleotide sequence of an endogenous gene and of the target nucleotide sequence.

Preferably said molecule is as defined above.

In a preferred embodiment, the molecule is for use in a real-time PCR or Droplet Digital PCR.

Another object of the invention is the use of the molecule as defined above to verify whether a PCR sample has contaminations and whether the PCR has been performed correctly and/or in *in vitro* or *ex vivo* diagnostic assays for the detection of one or more cancer-related genes, oncogenes, viral genes, bacterial genes, fungal genes or genes involved in pathologies selected from autoimmune diseases, inflammatory diseases, infectious diseases, metabolic disorders, degenerative diseases, neurological diseases, heart, bone, joint, muscle, brain, reproductive organs, blood, lymphatic tissues, glandular tissues, breast, digestive tract, respiratory tract or skin diseases.

A further object of the invention is a nucleic acid amplification kit comprising:
(a) the nucleic acid molecule to be used as the single control to ascertain the absence of contaminations and the operation of the reaction reagents as defined above;
(b) the detection mixture of said nucleic acid comprising a forward primer, a reverse primer and a specific probe, and
(c) a "master mix" comprising one or more DNA polymerases, triphosphate nucleotides, and optionally one or more reverse transcriptases; and
(d) a detection mixture for at least one target polynucleotide comprising one or more forward primers, one or more reverse primers and optionally one or more specific probes;
wherein (a), (b), (c) and (d) are single mixtures or are combined together in one or more mixtures or are combined together in a single mixture.

Another object of invention is a mixture of reagents for nucleotide amplification comprising:
(a) a "master mix" comprising one or more DNA polymerases, triphosphate nucleotides, and optionally one or more reverse transcriptases;
(b) a detection mixture for at least one target polynucleotide comprising one or more forward primers, one or more reverse primers and one or more specific probes; and
(c) a single control detection mixture to ascertain the absence of contaminations and the operation of the reaction reagents comprising a forward primer, a reverse primer and a specific probe capable of amplifying and detecting an amplifiable nucleic acid region comprised in said single control,

wherein (a), (b) and (c) are single mixtures or are combined together in one or more mixtures or are combined together in a single mixture and
where the single control is as defined above.

Furthermore, in the kit or in the mixture as above, the primers capable of triggering the PCR reaction of the single control are also capable of triggering the PCR reaction of the nucleotide sequence of an exogenous target and the kit or mixture further comprises a specific probe for the nucleotide sequence of said exogenous target

The kit and mixture according to the invention may detect one or more targets. For example, they may be used in a triplex or quadroplex type reaction.

The method of the invention allows to minimize the number of control reactions.

The method according to the invention allows to verify whether an analytical session of a PCR has contaminations and whether the PCR has been performed correctly.

The present authors have developed a new control to be used in PCR reactions called "Pan Control". Pan Control consists of a natural nucleic acid (DNA, RNA) or artificial nucleic acid (PNA, TNA, LNA or analogue). The nucleic acid is synthesized *in vitro* and inserted into a vector (plasmid, minigene, or any other type of vector) whose sequence is extraneous and/or partially extraneous to the desired targets, thus detection is ensured by the specific hybridization probe of the Pan Control alone.

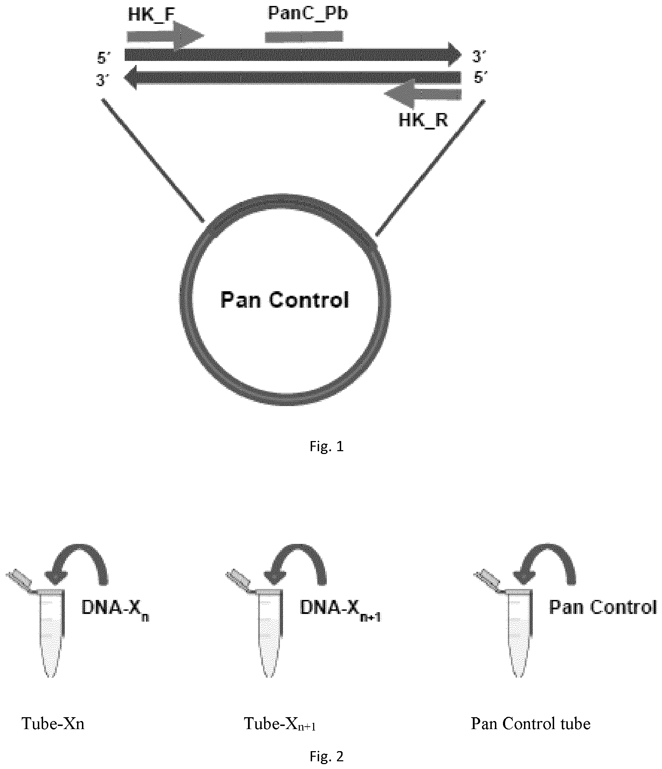

The amplifiable nucleotide sequence, contained in the Pan Control, has a minimum size in nucleotide bases (nucleotide bases is intended as Adenine, Thymine, Guanine, Cytosine, Uracil, Inosine or any other nucleotide analogue) of about 50 base pairs and contains two attachment sites for primers, respectively at the 5' and 3' ends and a complementary region internal to them for probe hybridization (Fig. 1). Said amplifiable nucleotide sequence preferably has a maximum length of about 200-500bp.

In a preferred embodiment, the qualitative or quantitative PCR reaction mixture contains:
- Specific primers which act as triggers for the amplification of one or more diagnostic targets;
- Specific probes which hybridize to one or more diagnostic targets;
- Specific primers which act as triggers for the amplification of the Pan Control;
- Specific probe which hybridizes to the amplifiable sequence of the Pan Control;
- Enzyme Taq polymerase;
- dNTPs;
- Reaction buffer.

The Pan Control is added in the qualitative or quantitative PCR session exclusively in the reaction tube dedicated to the control, replacing the usual positive (PC) and negative (NTC) control used (Fig. 2).

The Pan Control assay will give an amplification signal that can only be detected in the reaction tube to which it was added and will remain silent in all the other tubes.

The Pan Control is inserted in the reaction mixture at a predetermined concentration preferably ranging from a minimum of 10 copies/reaction to a maximum of 1 × 10⁶ copies/reaction.

The amplicon generated by the amplification of the Pan Control is identified by hybridization with the specific probe and detected in fluorescence or with any other colorimetric system.

In the absence of contamination and correct operation of the reaction, the expected results for the reaction tube of the Pan Control are as follows:
- No signal for the diagnostic targets present;
- Presence of signal for the Pan Control.

In the presence of contamination and correct operation of the reaction, the expected results for the reaction tube of the Pan Control are as follows:
- Presence of signal for at least one of the diagnostic targets present;
- Presence of signal for the Pan Control.

In the absence of contamination and incorrect operation of the reaction, the expected results for the reaction tube of the Pan Control are as follows:
- No signal for the diagnostic targets present;
- No signal for the Pan Control.

The present invention is applicable to any PCR assay, qualitative or quantitative, for research or diagnostics, using any type of chemistry known in the art.

In particular, the types of PCR in which the present invention may be used are for example:
- Qualitative PCR
- Absolute quantitative PCR
- Allelic discrimination
- Multiplex PCR
- Reverse transcription PCR
- High Resolution Melting (HRM)
- Melting analysis
- Relative Quantification

The sequence used as a control in the present invention preferably has at least one of the following characteristics:
- Inserted into a vector (plasmid, minigene, etc.);
- GC content of around 30-80%;
- Homopolymer sequence < 30 bp;
- Repeated sequence <20 bp for G/C;
- Repeated sequence <40 bp for A/T;
- Does not contain secondary hairpin type structure.

In the context of the present invention, "foreign sequence" or "different sequence" refers to a sequence completely different from the targets also at the primer attachment site level. "Partially foreign sequence" or "partially different sequence" refers to a sequence other than the targets except at the primer attachment site, where the sequence is complementary to the primers of one of the targets present in the PCR reaction.

Within the context of the present invention, an endogenous gene (or endogenous control or housekeeping gene) refers to a gene present in all the cells of the tested samples. For example, it refers to a gene constitutively expressed in all the analysed samples used to normalize data with respect to the amount of DNA loaded and to variations in reaction efficiency. Examples of endogenous genes are: β - globin, β - actin, β2 - microglobulin, Beta-glucuronidase (GUSB), ABL, RNase P, glyceraldehyde-3-phosphate dehydrogenase (GAPDH), Prothrombin, Methylenetrahydropolate reductase (MTHFR).

In the context of the present invention, the exogenous target (or exogenous control) is added to the sample to be tested and consists of a nucleotide sequence which may be derived from: Luciferase sequence, Tobacco mosaic virus sequence, Plant sequence such as that of a tomato, Lambda phage sequence or Equine viral arteritis sequence or it may consist of an artificial sequence. Said exogenous target is used to monitor the efficiency of the amplification and/or extraction reaction.

In the present invention the term "target nucleotide sequence" can be used interchangeably with "target polynucleotide".

In the present method the PCR conditions for the Pan Control must be the same as those applied to the target.

The nucleic acid sample subjected to the present method was obtained in any manner known to the person skilled in the art, e.g., by extraction or lysis of a sample, and from any cell source or tissue biopsy.

The method according to the invention comprises in one embodiment the reverse transcription of the nucleotide sequences of interest.

The probes and primers of the invention may be marked by any technique known to the person skilled in the art (e.g., fluorescent, radioactive or enzymatic markers). The detection of the primers and/or probes may be performed by any method known to the person skilled in the art, for example fluorescence detection, fluorescence quenching, colorimetric method, polarized fluorescence, chemiluminescence, FRET, electrochemiluminescence, and radioactivity.

As used herein, the term "hybridization" is used in reference to the coupling of complementary (including partially complementary) polynucleotide strands. The hybridization and the hybridization strength (i.e., the strength of the association between the polynucleotide strands) are characterized by many factors well known in the art, including the degree of complementarity between the polynucleotides, the stringency of conditions affected by, for example, salt concentration, the melting temperature of the forming hybrid, the presence of other compounds, the molarity of the strands to be hybridized, and the G:C content of the polynucleotide strands. An extensive guide to the hybridization of nucleic acids is found in Tijssen (1993) Laboratory Techniques in Biochemistry and Molecular Biology - Hybridization with Nucleic Acid Probes, Part I, Chapter 2 (Elsevier, New York); and Ausubel et al, eds. (1995) Current Protocols in Molecular Biology, Chapter 2 (Greene Publishing and Wiley-Interscience, New York). See Sambrook et al. (1989) Molecular Cloning: A Laboratory Manual (2d Ed., Cold Spring Harbor Laboratory Press, Plainview, New York). As discussed in more detail, the term "specific hybridization" refers to the hybridization of a polynucleotide, e.g., an oligonucleotide primer or probe or the like, to a target sequence, such as a sequence to be quantified in a sample, a positive control target nucleic acid sequence, or the like, and not to unrelated sequences, under conditions typically used for nucleic acid amplification. In certain embodiments, the primers and/or probes comprise oligonucleotides that hybridize to a target nucleic acid sequence along the entire length of the oligonucleotide sequence, or may form "mismatches" upon hybridization but retain the ability to hybridize under stringent or standard PCR conditions. For example, primers are provided in the present invention which comprise, consist essentially of, or are formed by a sequence selected from the group of SEQ ID NO: 2-11 or a variant thereof. In certain embodiments, an amplification primer set is provided, which may for example comprise one or more primer pairs. As used herein, the term "primer pair" or "two primers" may refer to two primers which individually hybridize to opposite strands of a target nucleic acid (e.g., the internal control sequences of a quantitative PCR), in which each primer may be extended to the 3' end thereof to form a target amplification product. The primer pairs may include forward and reverse primers. In some embodiments, the nucleic acid sequences provided herein are present within a vector, such as a plasmid. Vectors are well known in the art and a person skilled in the art will appreciate that many vectors can be used to provide a nucleic acid sequence. In addition, there are many variants of vectors and additional vectors may be produced by a person skilled in the art. The vectors can be modified by site-directed mutagenesis, random mutagenesis, or by one or more cloning steps to add, remove, and/or replace at least one nucleic acid sequence of the vector, e.g., a multiple cloning site, a resistance to gene antibiotics, an origin or replication, or a gene encoding a marker which facilitates the visualization of the vector carrier cells, such as beta galactosidase, luciferase, or green fluorescent protein. By way of example, in the present invention pEXA128-derived plasmid containing an internal control sequence is used.

The nucleic acids may be extracted from various types of samples, including biological or clinical samples such as tissue samples, body fluids or cell culture samples, as well as food samples, soil samples or the like, using a variety of techniques known in the art. The extraction of nucleic acid may comprise the separation of nucleic acids from other substances in a biological sample, e.g. proteins, lipids, membranes, organelles, carbohydrates and inorganic molecules. The nucleic acid extraction can be performed manually or by one of several automated systems.

In the context of the present invention, when reference is made to specific DNA sequences, it is intended that RNA molecules identical to said polynucleotides, except that the RNA sequence contains uracil instead of thymine and the skeleton of the RNA molecule contains ribose instead of deoxyribose, RNA sequences complementary to the sequences described therein, functional fragments, mutants and derivatives thereof, proteins encoded therein, functional fragments, mutants and derivatives thereof are also comprised in the invention.

The term "complementary" sequence refers to a polynucleotide which is not identical to the sequence but has a base sequence complementary to the first sequence or encodes the same amino acid sequence as the first sequence. A complementary sequence may comprise DNA and RNA polynucleotides.

The term "functional" may be understood as maintaining the same activity. The "fragments" are preferably at least 10 nt., 20 nt., 30 nt long.,...

The "derivatives" may be recombinant or synthetic. As used herein, the term "derivatives" also refers to longer or shorter polynucleotide sequences having, for example, a percentage identity of at least 80%, 85%, 86%, 87%, 88%, 89%, 90%, 91%, 92%, 93%, 94%, 95%, 96%, 97%, 98%, more preferably of at least 99%, with the sequences described herein.

In the present invention "at least 80% of identity" or "of homology" indicates that the identity (or homology) may be a sequence identity of at least 80%, or 85%, or 90%, or 95% or 100% with respect to the indicated sequences. This applies to all the above-mentioned identity percentages. In the present invention, "at least 90% of identity" or "of homology" indicates that the identity (or homology) may be a sequence identity of at least 90%, 91%, 92%, 93%, 94%, 95%, 96%, 97%, 98%, 99% or 100% with respect to the indicated sequences. This applies to all the above-mentioned identity percentages. Preferably, the identity percentage relates to the full length of the indicated sequence.

The alignment aimed at determining the amino acid sequence identity percentage can be achieved in various ways which are within the knowledge of the person skilled in the art, for instance, using publicly available computer software such as BLAST, BLAST-2, ALIGN or Megalign (DNASTAR) software. Also falling within the scope of the invention are polynucleotides having the same nucleotide sequences as a polynucleotide exemplified herein with the exception of nucleotide substitutions, additions or deletions within the polynucleotide sequence, as long as these variant polynucleotides substantially maintain the same relevant functional activity as the polynucleotides specifically exemplified herein. Thus, the polynucleotides described herein should be understood to comprise mutants, derivatives, variants and fragments, as discussed above, of the specifically exemplified sequences. The present invention also contemplates those polynucleotide molecules having sequences that are sufficiently homologous with the polynucleotide sequences of the invention to allow the hybridization with such sequence under stringent standard conditions and standard methods (Maniatis, T. et al, 1982). The polynucleotides described herein can also be defined in terms of more particular ranges of identity and/or similarity to those exemplified herein. The sequence identity will typically be greater than 60%, preferably greater than 75%, more preferably greater than 80%, even more preferably greater than 90%, and may be greater than 95%. The sequence identity and/or similarity may be 49, 50, 51, 52, 53, 54, 55, 56, 57, 58, 59, 60, 61, 62, 63, 64, 65, 66, 67, 68 , 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99% or greater with respect to a sequence exemplified herein. Also included in the present invention are nucleic acid sequences derived from the nucleotide sequences shown below, e.g., functional fragments, mutants, derivatives, analogs, and sequences with an identity percentage of at least 70% with the underlying sequences. In the mixture or sample according to the invention, the target polynucleotides comprise RNA or DNA polynucleotides. The DNA and RNA can be extracted by any known method. In the context of the present invention, polynucleotide or nucleic acid refers to an RNA, e.g., mRNA, DNA, e.g., genomic or derived from mRNA.

In the present invention, the primers and probes are designed or selected according to methods known to the person skilled in the art. The selected primers preferably meet at least one of the following requirements:
- Target sequence specificity
- Length in base pairs: 20-28 bp
- Amplicon size: 50 -150 bp
- Absence of dinucleotide repeats and guanosine rich sequences (G)
- Melting temperature (Tm) between 58°C and 60°C
- GC content between 50% and 60%
- Balance of domains rich of GC and AT
- Absence of intra-primer and inter-primer homology to avoid dimerization or self-dimer formation

The selected probe preferably meets at least one of the following requirements:
- Target gene sequence specificity
- Length in base pairs: 13 - 30 bp
- Designed in regions not involved in the formation of secondary structures
- Average melting temperature comprised between 68° and 70°C

In a preferred aspect of the invention, the target nucleotide sequence (or diagnostic target) to be identified is a viral or bacterial pathogen sequence, e.g., a human herpes virus type 8 (HHV 8) sequence.

Preferably, it is also identified in the present invention the target nucleotide sequence of an endogenous gene (or endogenous control), preferably housekeeping (HK assay), which allows to assess the possible presence of reaction inhibitors in extract and to monitor the extraction process.

The specific probes for the identification of amplicons are preferably marked with different fluorophores. The single control according to the present invention may be prepared by methods known to the person skilled in the art. It may for example be prepared as a plasmid, preferably a vector, comprising an insert containing a sequence partially extraneous to the assay targets using common genetic engineering techniques. In the present invention the single control is used, for example at a concentration of about 100 ng/µL. Typically, a stock solution with a concentration of about 10¹⁰ copies/ µL is used; then a serial dilution in TE buffer is carried out until the use solution with a concentration of 10³ copies/µL is obtained. The single control according to the invention may be amplified by primers of the HK assay and identified by a specific probe.

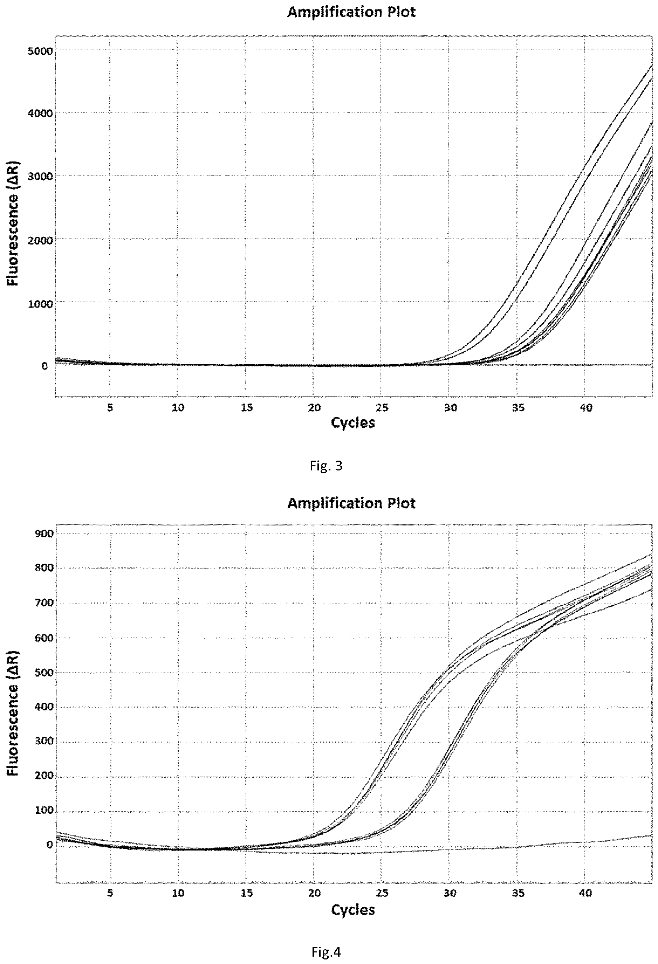

The present invention will now be illustrated by means of non-limiting examples, with reference to the following figures.
**Figure 1****.** Pan Control diagram. HK_F: forward Pan Control primer; HK_R: reverse Pan Control primer; PanC_Pb: specific Pan Control probe.
**Figure 2****.** Loading of samples and of Pan Control in a qualitative or quantitative PCR session.
Tube-Xn: tube in which the sample Xn is analysed; DNA-Xn: extracted DNA corresponds to sample Xn which is loaded into the reaction mixture; Tube -Xn+1: tube in which sample Xn+1 is analysed; DNA-Xn+1: extracted DNA corresponds to sample Xn+1 which is loaded into the reaction mixture; Pan Control tube: tube in which the Pan Control is loaded **Figure 3****:** CMV amplification curves (FAM fluorophore channel).
**Figure 4****:** HK gene amplifying curves (JOE fluorophore channel).

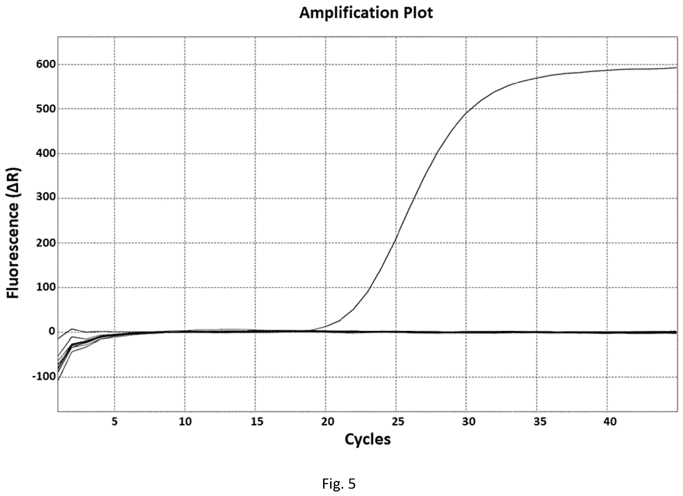

**Figure 5****:** Pan Control amplification curves (ROX fluorophore channel).

### DETAILED DESCRIPTION OF THE INVENTION

### Materials and Methods

For the design and analysis of the Pan Control primers and probes, the authors proceeded as follows:
1. Evaluation of possible gene sequences (in animal and plant fields) foreign or partially foreign to the targets normally studied in diagnostics and detection of possible primers and probes;
2. Design of primers and probes;
3. In silico analysis and validation of primers and probes.

The analysis and validation of primers and probes were performed using the following IT tools:
- RealTimeDesign^{™} (Biosearch Technologies) (http://www.biosearchtech.com/support/tools/design-software)
- Primer Express^{®} Software v3.0.1 (Thermo Scientific);
- Basic Local Alignment Search Tool (BLAST) (http://blast.ncbi.nlm.nih.gov/Blast.cgi). RealTimeDesign^{™} software was used to determine melting temperature, GC base content, amplicon length and likelihood of secondary structure formation.

Primer Express^{®} software was used for a further analysis of the identified sequences.

Primers and probes were also analysed with the tool BLAST to ensure the absence of unspecific pairings with other regions of the target sequence and especially with the human genome or any other pathogenic microorganism.

BLAST software can identify regions with similarities among sequences. The software can make a comparison of nucleotide or protein sequences with available databases and calculates the statistical significance of detected base matches.

The selected primers meet the following requirements:
- Target sequence specificity
- Length in base pairs: 20-28 bp
- Amplicon size: 50 -150 bp
- Absence of dinucleotide repeats and guanosine rich sequences (G)
- Melting temperature (Tm) between 58°C and 60°C
- GC content between 50% and 60%
- Balance of domains rich of GC and AT
- Absence of intra-primer and inter-primer homology to avoid dimerization or self-dimer formation

The selected probe meets the following requirements:
- Target gene sequence specificity
- Length in base pairs: 13 - 30 bp
- Designed in regions not involved in the formation of secondary structures
- Average melting temperature comprised between 68° and 70°C

Below is the sequence of the Pan Control insert and its primers (underlined letters) and probes (bold letters).

AAACCTGTCTTGTAACCTTGATACCAACCTGCCCAGGGCCTCACCACCAACTTCATCCACGTTCACCTAAACAATC
CGGAAGCGACCAACGCCTTGATTGACAAGGATGGATGGCTACATTCTGGAGACATAGCTTCTCCTCAGGAGTCA
GGTGCACCATGGTGTCTGTTTGAGGTTGCTAGTGAACACAGTTGTGT
(SEQ ID NO:1)

### Example 1 of Pan Control use in the assay for HHV 8 identification by real-time PCR

The test includes 3 different assays in a single reaction. In particular,
- primers and probes for the identification of human herpes virus type 8 (HHV 8),
- primers and probes for the amplification of a housekeeping gene (HK assay), which allows to evaluate the possible presence of reaction inhibitors in the extract and to monitor the extraction process;
- primers and probes for the detection of Pan Control.

The amplification reaction is set up as follows:

| **Components** | **Volume/Final concentration** |
|---|---|
| TaqMan^{™} Universal PCR Master Mix | 12.5 µL |
| HHV8_F | 250 µM |
| HHV8_R | 250 µM |
| HHV8_Pb | 250 µM |
| HK_F* | 200 µM |
| HK_R* | 200 µM |
| HK_Pb | 200 µM |
| PanC_Pb | 200 µM |
| H₂O | Sufficient quantity to bring to final volume of 20 µL |

| | |
|---|---|
| *in this case the primers of the HK assay are also used for the Pan Control assay. | |

The specific probes for the identification of the three different amplicons are marked with three different fluorophores which are detected in different channels of the real-time PCR instrument. The sequences of primers and probes with the indication of the respective markings are reported below:

| ID | Sequence | SEQ ID NOs |
|---|---|---|
| HHV8_F | CTCGAATCCAACGGATTTGAC | 2 |
| HHV8_R | GGCACGCTATTCTGCAGCA | 3 |
| HHV8_Pb | FAM - CCATGGTCGTGCCGCAGCA - BHQ1 | 4 |
| HK_F | CCTGTCTTGTAACCTTGATACCAA | 5 |
| HK_R | ATGGTGCAGCTGACTCCTGA | 6 |
| HK_Pb | JOE - ATCCACGTTCACCTTGCCCCACA - BHQ 1 | 7 |
| PanC_Pb | CAL Fluor Red 610 - ACGCCTTGATTGACAAGGATGGATGGc - BHQ2 | 8 |

The Pan Control is prepared by diluting the plasmid, consisting of a pEX-A128 vector (2450 bp, ampicillin antibiotic selective produced by Eurofins Genomics) and an insert containing a sequence partially extraneous to the assay targets, with H₂O or buffer. In particular, the lyophilic plasmid is resuspended in TE buffer in the volume necessary to obtain a concentration of 100 ng/µL. Considering the total length of Pan Control (insert + vector), considering that each nucleotide base has a molecular weight of 650D, that the Avogadro constant is equal to 6.022 × 10²³ mol⁻¹, the concentration in copies of Pan Control/µL is calculated. Normally this produces a stock solution which has a concentration of around 10¹⁰ copies/ µL. A serial dilution in TE buffer is then carried out until the use solution with a concentration of 10³ copies/µL is obtained. The Pan Control is amplified by the primers of the HK assay and identified by a specific probe (PanC_Pb; Fig. 1).

The tests are conducted on the AriaDx Real-Time PCR System (Agilent Technologies) instrument. The thermal profile is below:

| **Step** | **No. of cycles** | **Time** | **(°C)** |
|---|---|---|---|
| 1 | 1 | 02:00 | 50.0 |
| 2 | 1 | 10:00 | 95.0 |
| 3 | 45 | 00:15 | 95.0 |
| | | 01:00 | 60.0* |

| | | | |
|---|---|---|---|
| * Fluorescence collection step | | | |

The tests are performed by loading 10 µL of DNA extracted from whole blood samples (from a single donor and for which informed consent is available) that are negative or appropriately positivized with NATtrol^{™} Human Herpes Virus Type 8 Stock (ZeptoMetrix Corporation) and 10 µL of Pan Control into the reaction control well (Fig. 2). The samples and the Pan Control are amplified in single.

Below is a summary of the results in a suitable session:

| **Sample ID** | **Ct FAM** | **Ct JOE** | **Ct CF610** | **Result** |
|---|---|---|---|---|
| Pan Control | NEG | NEG | POS | Suitable control |
| 1 | POS | POS | NA | Positive for HHV-8 |
| 2 | NA | POS | NA | Negative for HHV-8 |

Below is a summary of the results in a session not suitable for HHV 8 contamination:

***The session is invalidated. The samples are automatically unsuitable as the PanControl also presents a signal in the FAM fluorophore channel (HHV 8 contamination) in addition to the specific signal.**

| **Sample ID** | **Ct FAM** | **Ct JOE** | **Ct CF610** | **Result** |
|---|---|---|---|---|
| Pan Control | POS | NEG | POS | Unsuitable control |
| 1 | POS | POS | NEG | Unsuitable* |
| 2 | NEG | POS | NEG | Unsuitable* |

Below is the summary of the results in an unsuitable session due to contamination by the housekeeping gene:

| **Sample ID** | **Ct FAM** | **Ct JOE** | **Ct CF610** | **Result** |
|---|---|---|---|---|
| Pan Control | NEG | POS | POS | Unsuitable control |
| 1 | POS | POS | NEG | Unsuitable* |
| 2 | NEG | POS | NEG | Unsuitable* |

| | | | | |
|---|---|---|---|---|
| *The session is invalidated. The samples are automatically unsuitable as the Pan Control also presents a signal in the JOE fluorophore channel (housekeeping gene contamination) in addition to the specific signal. | | | | |

Below is a summary of the results in an unsuitable session due to reaction malfunction:

| **Sample ID** | **Ct FAM** | **Ct JOE** | **Ct CF610** | **Result** |
|---|---|---|---|---|
| Pan Control | NEG | NEG | NEG | Unsuitable control |
| 1 | POS | POS | NEG | Unsuitable* |
| 2 | NEG | POS | NEG | Unsuitable* |

| | | | | |
|---|---|---|---|---|
| *The session is invalidated. The samples are automatically unsuitable due to a lack of the Pan Control signal. | | | | |

### Example 2

The test described below was developed for the quantification of Cytomegalovirus (CMV) in DNA extracted from clinical samples including whole blood in real-time PCR.

The master mix of the CMV quantification test contains the following components:
- Real-time PCR reaction buffer
- Primer and probe marked with FAM fluorophore for the CMV amplification
- Primer and probe marked with the JOE fluorophore for the amplification of a housekeeping gene (HK assay), which allows to evaluate the possible presence reaction inhibitor in the extract and to monitor the extraction process;
- Probe marked with the ROX fluorophore for the detection of the Pan Control.

The specific probes for the identification of the three different target amplicons are marked with three different fluorophores which are detected in three different channels of the real-time PCR instrument.

The Pan Control and primers and probes used for detecting the HK gene and the Pan Control used are the same as those described in example 1.

The sequences of primers and probe with the indication marking for the CMV amplification are reported below:

| ID | Sequence | SEQ ID NOs |
|---|---|---|
| CMV_F | CCACACTAGGAGAGCAGACT | 9 |
| CMV_R | GCCAAGCGGCCTCTGAT | 10 |
| CMV_Pb | FAM - GCATGAAGGTCTTTGCCC-BHQ1 | 11 |

The tests are conducted on the AriaDx Real-Time PCR System (Agilent Technologies) instrument. The thermal profile is below:

| **Step** | **No. of cycles** | **Time** | **(°C)** |
|---|---|---|---|
| 1 | 1 | 02:00 | 50.0 |
| 2 | 1 | 10:00 | 95.0 |
| 3 | 45 | 00:15 | 95.0 |
| | | 01:00 | 60.0* |

| | | | |
|---|---|---|---|
| * Fluorescence collection step | | | |

The amplification reaction is set up by mixing 20 µL of master mix with 10 µL of extracted DNA. The test was performed by testing DNA from an external quality control panel for CMV consisting of 10 CMV-positive or negative,frozen blood samples with different viral loads. The test was performed using a 96-well real-time PCR plate. During the plate preparation step, 20 µL of master mix were aliquoted into 15 different wells. In the first 10 wells (A1-H1 and A2-B2) 10 µL of DNA from the external quality control samples were added, then in the next 4 wells the CMV quantification standard (C2-F2) and in the last well (position G2) 10 µL of Pan Control were added. (Table 1)

**Table 1. Real-time PCR plate preparation diagram**

| | **1** | **2** | **3** | **4** | **5** | **6** | **7** | **8** | **9** | **10** | **11** | **12** |
|---|---|---|---|---|---|---|---|---|---|---|---|---|
| **A** | CMV-01 | CMV - 09 | | | | | | | | | | |
| **B** | CMV - 02 | CMV - 10 | | | | | | | | | | |
| **C** | CMV -03 | STD 10²c/rx | | | | | | | | | | |
| **D** | CMV - 04 | STD 10³c/rx | | | | | | | | | | |
| **E** | CMV - 05 | STD 10⁴c/rx | | | | | | | | | | |
| **F** | CMV -06 | STD 10⁵c/rx | | | | | | | | | | |
| **G** | CMV - 07 | Pan Control | | | | | | | | | | |
| **H** | CMV -08 | | | | | | | | | | | |

The results analysis shows that the session is suitable given that the Pan Control correctly produces only one signal in the ROX channel, indicating the suitability of the amplification reaction and the exclusion of possible contamination phenomena.

Below the detailed results obtained:

| **Well** | **SAMPLE NAME** | **Ct FAM (CMV)** | **Ct JOE (IC)** | **Ct ROX (Pan Control)** | **EXPECTED RESULT (Log₁₀ copies/mL)** | **RESULT OBTAINED (Log₁₀ copies/mL)** |
|---|---|---|---|---|---|---|
| G2 | Pan Control | N/A | N/A | 21.25 | - | - |
| A1 | CMV - 01 | 35.94 | 24.74 | N/A | 2.79 | 2.77 |
| B1 | CMV - 02 | 31.19 | 25.1 | N/A | 3.94 | 4.17 |
| C1 | CMV - 03 | 35.8 | 25.14 | N/A | 2.60 | 2.82 |
| D1 | CMV - 04 | 35.16 | 24.95 | N/A | 2.80 | 3.00 |
| E1 | CMV - 05 | 34.63 | 24.87 | N/A | 3.05 | 3.16 |
| F1 | CMV - 06 | 36.18 | 20.3 | N/A | 3.05 | 2.70 |
| G1 | CMV - 07 | 35.66 | 20.4 | N/A | 3.06 | 2.86 |
| H1 | CMV - 08 | 31.99 | 20.25 | N/A | 4.30 | 3.94 |
| A2 | CMV - 09 | N/A | 20.62 | N/A | CMV Negative | CMV Negative |
| B2 | CMV - 10 | 36.33 | 19.94 | N/A | 2.95 | 2.66 |

The amplification curve graphs in the channels of the three fluorophores considered are shown in figures 3, 4 and 5.

Similar results were also obtained in an allelic discrimination assay, where prothrombin or MTHFR was used as the endogenous gene and the same primer pair for the PCR reaction of the target nucleotide sequence (protothrombin or MTHFR), for the PCR reaction of the single control and for the endogenous gene were used in addition to a single control adapted to the sequence.

## Claims

1. An *ex vivo* or *in vitro* method for carrying out an amplification of at least one target nucleotide sequence by a polymerase chain reaction (PCR), comprising the following steps:
a) providing a first environment comprising at least one sample possibly containing said target nucleotide sequence;
b) providing a second environment comprising a single control to ascertain the absence of contamination and the functioning of the reaction reagents,
said single control containing a nucleotide sequence comprising:
- an amplifiable nucleic acid having a different or partially different sequence from said target nucleotide sequence and
- two hybridization sites for two primers, respectively at the 5 'and 3' ends of said amplifiable nucleic acid, and
- a region inside the primer hybridization sites for the hybridization of a probe having a different sequence from said target nucleotide sequence,
c) adding a mixture to said sample and to said single control comprising:
- two primers capable of triggering the PCR reaction of the target nucleotide sequence;
- two primers capable of triggering the PCR reaction of the single control;
- a first specific probe for the target nucleotide sequence;
- a second specific probe for the amplifiable sequence of the single control;
- appropriate reagents;
d) performing the PCR reaction on said mixture to obtain and detect copies of the target nucleotide sequence if present in said sample and copies of the nucleotide sequence of the single control,
wherein the two primers capable of triggering the PCR reaction of the single control are also capable of triggering the PCR reaction of the nucleotide sequence of an exogenous target previously added to the sample of step a
and wherein preferably in step c) the mixture further comprises a third specific probe for the nucleotide sequence of said exogenous target.

2. The method according to claim 1, wherein the probes are marked with fluorophores different from one another.

3. The method according to one of the preceding claims, wherein said single control consists of a vector or a linear DNA strand, preferably of at least 50 nucleotides and/or
wherein the amplifiable nucleic acid contained in the single control has at least one of the following characteristics:
- GC content of around 30-80%;
- Sequence of homopolymers with a length less than 30 bp;
- Repeated sequence with a length less than 20 bp for G/C;
- Repeated sequence with a length less than 40 bp for A/T;
- Does not contain secondary hairpin type structure and/or
wherein the single control comprises or consists of a sequence having an identity of at least 80% with the sequence of SEQ ID NO: 1 and/or
wherein said mixture comprises a forward primer comprising a sequence having an identity of at least 80% with the sequence CCTGTCTTGTAACCTTGATACCAA (SEQ ID NO: 5), a reverse primer comprising a sequence having an identity of at least 80% with the sequence ATGGTGCAGCTGACTCCTGA (SEQ ID NO: 6) and a probe comprising a sequence having an identity of at least 80% with the sequence ACGCCTTGATtgACAAGGATGGATGGc (SEQ ID NO: 8).

4. The method according to one of the preceding claims, wherein said method is a diagnostic method, for example for the detection of one or more cancer-related genes, oncogenes, viral genes, bacterial genes, fungal genes or genes involved in pathologies selected from autoimmune diseases, inflammatory diseases, infectious diseases, metabolic disorders, degenerative diseases, neurological diseases, heart, bone, joint, muscle, brain, reproductive organs, blood, lymphatic tissues, glandular tissues, breast, digestive tracts, respiratory tract or skin diseases
and/or wherein the PCR is a qualitative PCR, absolute quantitative PCR, allelic discrimination, multiplex PCR, Reverse transcription PCR, High Resolution Melting (HRM), Melting analysis, Relative quantification, preferably a RT-PCR, a real-time PCR, Droplet Digital PCR or a quantitative real-time RT-PCR.

5. The method according to claim 4, wherein the PCR is a real-time PCR or Droplet Digital PCR.

6. Use of a nucleic acid molecule to be used as a single control in a polymerase chain reaction (PCR) in the method of any one of claims 1-5, said single control comprising a nucleotide sequence comprising:
- an amplifiable nucleic acid having a different or partially different sequence from said target nucleotide sequence,
- two hybridization sites for two primers, respectively at the 5 'and 3' ends of said amplifiable nucleic acid, and
- a region inside the primer hybridization sites for the hybridization of a probe having a different sequence from said target nucleotide sequence.

7. Use of the molecule according to claim 6, wherein the hybridization sites for primers comprise a sequence with a homology percentage of at least 90% with respect to the sequence of an endogenous gene or an exogenous target to be used as controls and/or the target nucleotide sequence.

8. Use of the molecule according to claim 6 or 7, as defined in claim 3, preferably wherein the PCR is real-time PCR or Droplet Digital PCR.

9. Use of the molecule according to any one of claims 6-8 to verify whether a PCR sample presents contaminations and whether the PCR has been performed correctly and/or in in vitro or ex vivo diagnostic assays for the detection of one or more cancer-related genes, oncogenes, viral genes, bacterial genes, fungal genes or genes involved in pathologies selected from autoimmune diseases, inflammatory diseases, infectious diseases, metabolic disorders, degenerative diseases, neurological diseases, heart, bone, joint, muscle, brain, reproductive organs, blood, lymphatic tissues, glandular tissues, breast, digestive tract, respiratory tract or skin diseases.

10. A nucleic acid amplification kit comprising:
(a) the nucleic acid molecule to be used as the single control to ascertain the absence of contaminations and the operation of the reaction reagents as defined in any one of claims 1 and 3 and 6-8;
(b) the detection mixture of said nucleic acid comprising a forward primer, a reverse primer and a specific probe, and
(c) a "master mix" comprising one or more DNA polymerases, triphosphate nucleotides, and optionally one or more reverse transcriptases; and
(d) a detection mixture for at least one target polynucleotide comprising one or more forward primers, one or more reverse primers and optionally one or more specific probes;
wherein (a), (b), (c) and (d) are single mixtures or are combined together in one or more mixtures or are combined together in a single mixture
wherein the single control primers are also capable of triggering the PCR reaction of the nucleotide sequence of an exogenous target
and wherein the kit further comprises a specific probe for the nucleotide sequence of said exogenous target.

11. A mixture of reagents for nucleotide amplification comprising:
(a) a "master mix" comprising one or more DNA polymerases, triphosphate nucleotides, and optionally one or more reverse transcriptases;
(b) a detection mixture for at least one target polynucleotide comprising one or more forward primers, one or more reverse primers and one or more specific probes; and
(c) a single control detection mixture to ascertain the absence of contaminations and the operation of the reaction reagents comprising a forward primer, a reverse primer and a specific probe capable of amplifying and detecting an amplifiable nucleic acid region comprised in said single control,
wherein (a), (b) and (c) are single mixtures or are combined together in one or more mixtures or are combined together in a single mixture and
wherein the single control is as defined in one of claims 1, 3 and 6-8
wherein the single control primers are also capable of triggering the PCR reaction of the nucleotide sequence of an exogenous target
and wherein the mixture further comprises a specific probe for the nucleotide sequence of said exogenous target.

## Patentansprüche

1. Ex vivo- oder in vitro-Verfahren zur Durchführung einer Amplifikation von mindestens einer Target-Nukleotidsequenz durch eine Polymerase-Kettenreaktion (PCR), umfassend die folgenden Schritte:
a) Bereitstellen einer ersten Umgebung, umfassend mindestens eine Probe, die potenziell die Target-Nukleotidsequenz enthält;
b) Bereitstellen einer zweiten Umgebung, umfassend eine einzelne Kontrolle, um das Nichtvorhandensein von Verunreinigungen und das Funktionieren der Reaktionsreagenzien festzustellen,
wobei die einzelne Kontrolle eine Nukleotidsequenz enthält, die umfasst:
- eine amplifizierbare Nukleinsäure mit einer unterschiedlichen oder partiell unterschiedlichen Sequenz von der Target-Nukleotidsequenz, und
- zwei Hybridisierungsstellen für zwei Primer, jeweils an dem 5'- und dem 3'-Ende der amplifizierbaren Nukleinsäure, und
- eine Region innerhalb der Primer-Hybridisierungsstellen für die Hybridisation einer Sonde mit einer unterschiedlichen Sequenz von der Target-Nukleotidsequenz,
c) Zugeben eines Gemischs zu der Probe und zu der einzelnen Kontrolle, umfassend:
- zwei Primer, die in der Lage sind, die PCR-Reaktion der Target-Nukleotidsequenz hervorzurufen;
- zwei Primer, die in der Lage sind, die PCR-Reaktion der einzelnen Kontrolle hervorzurufen;
- eine erste spezifische Sonde für die Target-Nukleotidsequenz;
- eine zweite spezifische Sonde für die amplifizierbare Sequenz der einzelnen Kontrolle;
- geeignete Reagenzien;
d) Durchführen der PCR-Reaktion an dem Gemisch, um Kopien der Target-Nukleotidsequenz, wenn in der Probe vorhanden, und Kopien der Nukleotidsequenz der einzelnen Kontrolle zu erhalten und nachzuweisen,
wobei die beiden Primer, die zum Hervorrufen der PCR-Reaktion der einzelnen Kontrolle in der Lage sind,
auch zum Hervorrufen der PCR-Reaktion der Nukleotidsequenz eines exogenen Targets, das zuvor zu der Probe aus Schritt a) gegeben wurde, in der Lage sind,
und wobei vorzugsweise in Schritt c) das Gemisch außerdem eine dritte spezifische Sonde für die Nukleotidsequenz des exogenen Targets umfasst.

2. Das Verfahren gemäß Anspruch 1, wobei die Sonden mit voneinander verschiedenen Fluorophoren markiert sind.

3. Das Verfahren gemäß einem der vorangehenden Ansprüche, wobei die einzelne Kontrolle aus einem Vektor oder einem linearen DNA-Strang, vorzugsweise aus mindestens 50 Nukleotiden, besteht und/oder
wobei die amplifizierbare Nukleinsäure, die in der einzelnen Kontrolle enthalten ist, mindestens eines der folgenden Merkmale aufweist:
- GC-Gehalt von etwa 30 - 80 %;
- Sequenz von Homopolymeren mit einer Länge von weniger als 30 bp;
- Wiederholungssequenz mit einer Länge von weniger als 20 bp für G/C;
- Wiederholungssequenz mit einer Länge von weniger als 40 bp für A/T;
- Enthält keine sekundäre Haarnadelstruktur und/oder
wobei die einzelne Kontrolle eine Sequenz mit einer Identität von mindestens 80 % mit der Sequenz von SEQ ID No. 1 umfasst oder daraus besteht, und/oder wobei das Gemisch einen Vorwärtsprimer, umfassend eine Sequenz mit einer Identität von mindestens 80 % mit der Sequenz CCTGTCTTGTAACCTTGATACCAA (SEQ ID No. 5), einen Rückwärtsprimer, umfassend eine Sequenz mit einer Identität von mindestens 80 % mit der Sequenz ATGGTGCAGCTGACTCCTGA (SEQ ID No. 6), und eine Sonde, umfassend eine Sequenz mit einer Identität von mindestens 80 % mit der Sequenz ACGCCTTGATtgACAAGGATGGATGGc (SEQ ID No. 8), umfasst.

4. Das Verfahren gemäß einem der vorangehenden Ansprüche, wobei das Verfahren ein diagnostisches Verfahren ist, zum Beispiel für den Nachweis eines oder mehrerer krebsassoziierter Gene, Onkogene, viralen Gene, bakteriellen Gene, fungalen Gene oder Gene, die an Pathologien, ausgewählt aus Autoimmunerkrankungen, Entzündungserkrankungen, Infektionserkrankungen, Stoffwechselstörungen, degenerativen Erkrankungen, neurologischen Erkrankungen, Erkrankungen von Herz, Knochen, Gelenken, Muskeln, Gehirn, Fortpflanzungsorganen, Blut, lymphatischen Geweben, Drüsengeweben, Brust, Verdauungstrakt, Atemwegstrakt oder der Haut, beteiligt sind,
und/oder wobei die PCR eine qualitative PCR, absolute quantitative PCR, Allelunterscheidung, Multiplex-PCR, Reverse Transkriptions-PCR, Hochauflösende Schmelze (HRM), Schmelzanalyse, Relative Quantifizierung, vorzugsweise eine RT-PCR, eine Echtzeit-PCR, eine Digitale Tröpfchen-PCR oder eine quantitative Echtzeit-RT-PCR ist.

5. Das Verfahren gemäß Anspruch 4, wobei die PCR eine Echtzeit-PCR oder eine Digitale Tröpfchen-PCR ist.

6. Verwendung eines Nukleinsäuremoleküls zur Benutzung als einer einzelnen Kontrolle in einer Polymerase-Kettenreaktion (PCR) bei dem Verfahren nach einem der Ansprüche 1 - 5, wobei die einzelne Kontrolle eine Nukleotidsequenz umfasst, die beinhaltet:
- eine amplifizierbare Nukleinsäure mit einer unterschiedlichen oder partiell unterschiedlichen Sequenz von der Target-Nukleotidsequenz,
- zwei Hybridisierungsstellen für zwei Primer, jeweils an dem 5'- und dem 3'-Ende der amplifizierbaren Nukleinsäure, und
- eine Region innerhalb der Primer-Hybridisierungsstellen für die Hybridisation einer Sonde mit einer unterschiedlichen Sequenz von der Target-Nukleotidsequenz.

7. Verwendung des Moleküls gemäß Anspruch 6, wobei die Hybridisierungsstellen für Primer eine Sequenz mit einer prozentualen Homologie von mindestens 90 % in Bezug auf die Sequenz eines endogenen Gens oder eines exogenen Targets, die als Kontrollen und/oder der Target-Nukleotidsequenz verwendet werden sollen, umfasst.

8. Verwendung des Moleküls gemäß Anspruch 6 oder 7, wie in Anspruch 3 definiert, vorzugsweise wobei die PCR eine Echtzeit-PCR oder eine Digitale Tröpfchen-PCR ist.

9. Verwendung des Moleküls gemäß einem der Ansprüche 6 - 8, um zu verifizieren, ob eine PCR-Probe Verunreinigungen aufweist und ob die PCR korrekt durchgeführt worden ist, und/oder in diagnostischen in vitro- oder ex vivo-Assays für den Nachweis eines oder mehrerer krebsassoziierter Gene, Onkogene, viralen Gene, bakteriellen Gene, fungalen Gene oder Gene, die an Pathologien, ausgewählt aus Autoimmunerkrankungen, Entzündungserkrankungen, Infektionserkrankungen, Stoffwechselstörungen, degenerativen Erkrankungen, neurologischen Erkrankungen, Erkrankungen von Herz, Knochen, Gelenken, Muskeln, Gehirn, Fortpflanzungsorganen, Blut, lymphatischen Geweben, Drüsengeweben, Brust, Verdauungstrakt, Atemwegstrakt oder der Haut, beteiligt sind.

10. Nukleinsäure-Amplifikationskit, umfassend:
(a) das Nukleinsäuremolekül zur Verwendung als einer einzelnen Kontrolle, um das Nichtvorhandensein von Verunreinigungen und das Funktionieren der Reaktionsreagenzien, wie in einem der Ansprüche 1 und 3 und 6 - 8 definiert, festzustellen;
(b) das Nachweisgemisch aus der Nukleinsäure, umfassend einen Vorwärtsprimer, einen Rückwärtsprimer und eine spezifische Sonde, und
(c) einen "Mastermix", umfassend eine oder mehrere DNA-Polymerasen, Triphosphat-Nukleotide und optional eine oder mehrere reverse Transkriptasen; und
(d) ein Nachweisgemisch für mindestens ein Target-Polynukleotid, umfassend einen oder mehrere Vorwärtsprimer, einen oder mehrere Rückwärtsprimer und optional eine oder mehrere spezifische Sonden;
wobei (a), (b), (c) und (d) einzelne Gemische sind oder in ein oder mehreren Gemischen untereinander kombiniert sind oder in einer Einzelmischung miteinander kombiniert sind,
wobei die Primer der einzelnen Kontrolle ebenfalls in der Lage sind, die PCR-Reaktion der Nukleotidsequenz eines exogenen Targets hervorzurufen,
und wobei das Kit weiterhin eine spezifische Sonde für die Nukleotidsequenz des exogenen Targets umfasst.

11. Gemisch aus Reagenzien für die Nukleotidamplifikation, umfassend:
(a) einen "Mastermix", umfassend eine oder mehrere DNA-Polymerasen, Triphosphat-Nukleotide und optional eine oder mehrere reverse Transkriptasen;
(b) ein Nachweisgemisch für mindestens ein Target-Polynukleotid, umfassend einen oder mehrere Vorwärtsprimer, einen oder mehrere Rückwärtsprimer und eine oder mehrere spezifische Sonden; und
(c) ein Einzelkontrolle-Nachweisgemisch, um das Nichtvorhandensein von Verunreinigungen und das Funktionieren der Reaktionsreagenzien festzustellen, umfassend einen Vorwärtsprimer, einen Rückwärtsprimer und eine spezifische Sonde, das in der Lage ist, eine amplifizierbare Nukleinsäureregion, die in der einzelnen Kontrolle enthalten ist, zu amplifizieren und nachzuweisen,
wobei (a), (b) und (c) einzelne Gemische sind oder zu ein oder mehreren Gemischen untereinander kombiniert sind oder in einer Einzelmischung miteinander kombiniert sind, und
wobei die einzelne Kontrolle wie in einem der Ansprüche 1, 3 und 6 - 8 definiert ist,
wobei die Primer der einzelnen Kontrolle ebenfalls in der Lage sind, die PCR-Reaktion der Nukleotidsequenz eines exogenen Targets hervorzurufen,
und wobei das Gemisch weiterhin eine spezifische Sonde für die Nukleotidsequenz des exogenen Targets umfasst.

## Revendications

1. Méthode ex *vivo* ou *in vitro* permettant de réaliser une amplification d'au moins une séquence nucléotidique cible par une réaction en chaîne par polymérase (PCR), comprenant les étapes suivantes :
a) fournir un premier environnement comprenant au moins un échantillon contenant éventuellement ladite séquence nucléotidique cible ;
b) fournir un second environnement comprenant un contrôle unique pour s'assurer de l'absence de contamination et du fonctionnement des réactifs réactionnels,
ledit contrôle unique contenant une séquence nucléotidique comprenant :
- un acide nucléique amplifiable ayant une séquence différente ou partiellement différente de ladite séquence nucléotidique cible et
- deux sites d'hybridation pour deux amorces, respectivement aux extrémités 5' et 3' dudit acide nucléique amplifiable, et
- une région à l'intérieur des sites d'hybridation des amorces pour l'hybridation d'une sonde ayant une séquence différente de ladite séquence nucléotidique cible,
c) ajouter un mélange audit échantillon et audit contrôle unique comprenant :
- deux amorces capables de déclencher la réaction PCR de la séquence nucléotidique cible ;
- deux amorces capables de déclencher la réaction PCR du contrôle unique ;
- une première sonde spécifique pour la séquence nucléotidique cible ;
- une deuxième sonde spécifique pour la séquence amplifiable du contrôle unique ;
- des réactifs appropriés ;
d) effectuer la réaction PCR sur ledit mélange pour obtenir et détecter des copies de la séquence nucléotidique cible si elle est présente dans ledit échantillon et des copies de la séquence nucléotidique du contrôle unique,
dans laquelle les deux amorces capables de déclencher la réaction PCR du contrôle unique sont également capables de déclencher la réaction PCR de la séquence nucléotidique d'une cible exogène préalablement ajoutée à l'échantillon de l'étape a,
et dans laquelle, de préférence à l'étape c), le mélange comprend en outre une troisième sonde spécifique pour la séquence nucléotidique de ladite cible exogène.

2. Méthode selon la revendication 1, dans laquelle les sondes sont marquées avec des fluorophores différents les uns des autres.

3. Méthode selon l'une des revendications précédentes, dans laquelle ledit contrôle unique est constitué d'un vecteur ou d'un brin d'ADN linéaire, de préférence d'au moins 50 nucléotides et/ou
dans laquelle l'acide nucléique amplifiable contenu dans le contrôle unique présente au moins l'une des caractéristiques suivantes :
- teneur en GC d'environ 30 à 80 % ;
- séquence d'homopolymères ayant une longueur inférieure à 30 pb ;
- séquence répétée ayant une longueur inférieure à 20 pb pour G/C ;
- séquence répétée ayant une longueur inférieure à 40 pb pour A/T ;
- ne contient pas de structure secondaire de type épingle à cheveux et/ou
dans laquelle le contrôle unique comprend ou est constitué d'une séquence ayant une identité d'au moins 80 % avec la séquence de SEQ ID NO : 1 et/ou dans laquelle ledit mélange comprend une amorce sens comprenant une séquence ayant une identité d'au moins 80 % avec la séquence CCTGTCTTGTAACCTTGATACCAA (SEQ ID NO : 5), une amorce antisens comprenant une séquence ayant une identité d'au moins 80 % avec la séquence ATGGTGCAGCTGACTCCTGA (SEQ ID NO : 6) et une sonde comprenant une séquence présentant une identité d'au moins 80 % avec la séquence ACGCCTTGATtgACAAGGATGGATGGc (SEQ ID NO : 8).

4. Méthode selon l'une des revendications précédentes, dans laquelle ladite méthode est une méthode de diagnostic, par exemple pour la détection d'un ou plusieurs gènes liés à un cancer, oncogènes, gènes viraux, gènes bactériens, gènes fongiques ou gènes impliqués dans des pathologies sélectionnées parmi les maladies auto-immunes, les maladies inflammatoires, les maladies infectieuses, les troubles métaboliques, les maladies dégénératives, les maladies neurologiques, les maladies du coeur, des os, des articulations, des muscles, du cerveau, des organes reproducteurs, du sang, des tissus lymphatiques, des tissus glandulaires, du sein, des voies digestives, des voies respiratoires ou de la peau
et/ou dans laquelle la PCR est une PCR qualitative, une PCR quantitative absolue, une discrimination allélique, une PCR multiplex, une PCR de transcription inverse, une fusion haute résolution (FHR), une analyse de fusion, une quantification relative, de préférence une RT-PCR, une PCR en temps réel, une PCR digitale en gouttelettes ou une RT-PCR quantitative en temps réel.

5. Procédé selon la revendication 4, dans laquelle la PCR est une PCR en temps réel ou une PCR digitale en gouttelettes.

6. Utilisation d'une molécule d'acide nucléique destinée à utiliser comme contrôle unique dans une réaction en chaîne par polymérase (PCR) dans la méthode selon l'une quelconque des revendications 1 à 5, ledit contrôle unique comprenant une séquence nucléotidique comprenant :
- un acide nucléique amplifiable ayant une séquence différente ou partiellement différente de ladite séquence nucléotidique cible,
- deux sites d'hybridation pour deux amorces, respectivement aux extrémités 5' et 3' dudit acide nucléique amplifiable, et
- une région à l'intérieur des sites d'hybridation des amorces pour l'hybridation d'une sonde ayant une séquence différente de ladite séquence nucléotidique cible.

7. Utilisation de la molécule selon la revendication 6, dans laquelle les sites d'hybridation pour les amorces comprennent une séquence avec un pourcentage d'homologie d'au moins 90 % par rapport à la séquence d'un gène endogène ou d'une cible exogène à utiliser comme contrôles et/ou la cible séquence nucléotidique.

8. Utilisation de la molécule selon la revendication 6 ou 7, telle que définie dans la revendication 3, de préférence dans laquelle la PCR est une PCR en temps réel ou une PCR digitale en gouttelettes.

9. Utilisation de la molécule selon l'une quelconque des revendications 6 à 8 pour vérifier si un échantillon PCR présente des contaminations et si la PCR a été effectuée correctement et/ou dans des tests de diagnostic in vitro ou ex vivo pour la détection d'un ou plusieurs gènes liés à un cancer, oncogènes, gènes viraux, gènes bactériens, gènes fongiques ou gènes impliqués dans des pathologies sélectionnées parmi les maladies auto-immunes, les maladies inflammatoires, les maladies infectieuses, les troubles métaboliques, les maladies dégénératives, les maladies neurologiques, les maladies du coeur, des os, des articulations, des muscles, du cerveau, des organes reproducteurs, du sang, des tissus lymphatiques, des tissus glandulaires, du sein, des voies digestives, des voies respiratoires ou de la peau.

10. Kit d'amplification d'acide nucléique comprenant :
(a) la molécule d'acide nucléique à utiliser comme contrôle unique pour vérifier l'absence de contaminations et le fonctionnement des réactifs de réaction comme défini dans l'une quelconque des revendications 1 et 3 et 6 à 8 ;
(b) le mélange de détection dudit acide nucléique comprenant une amorce sens, une amorce antisens et une sonde spécifique, et
(c) un « mélange maître » comprenant une ou plusieurs ADN polymérases, des triphosphate nucléotides et éventuellement une ou plusieurs transcriptases inverses ; et
(d) un mélange de détection pour au moins un polynucléotide cible comprenant une ou plusieurs amorces sens, une ou plusieurs amorces antisens et éventuellement une ou plusieurs sondes spécifiques ;
dans lequel (a), (b), (c) et (d) sont des mélanges uniques ou sont combinés ensemble dans un ou plusieurs mélanges ou sont combinés ensemble dans un mélange unique
dans lequel les amorces de contrôle unique sont également capables de déclencher la réaction PCR de la séquence nucléotidique d'une cible exogène et dans lequel le kit comprend en outre une sonde spécifique pour la séquence nucléotidique de ladite cible exogène.

11. Mélange de réactifs pour l'amplification de nucléotides comprenant :
(a) un « mélange maître » comprenant une ou plusieurs ADN polymérases, des triphosphate nucléotides et éventuellement une ou plusieurs transcriptases inverses ;
(b) un mélange de détection pour au moins un polynucléotide cible comprenant une ou plusieurs amorces sens, une ou plusieurs amorces antisens et éventuellement une ou plusieurs sondes spécifiques ;et
(c) un mélange de détection de contrôle unique pour vérifier l'absence de contaminations et le fonctionnement des réactifs de réaction comprenant une amorce sens, une amorce antisens et une sonde spécifique capable d'amplifier et de détecter une région d'acide nucléique amplifiable comprise dans ledit contrôle unique,
dans lequel (a), (b) et (c) sont des mélanges uniques ou sont combinés ensemble dans un ou plusieurs mélanges ou sont combinés ensemble dans un mélange unique et dans lequel le contrôle unique est tel que défini dans l'une des revendications 1, 3 et 6 à 8 dans lequel les amorces de contrôle unique sont également capables de déclencher la réaction PCR de la séquence nucléotidique d'une cible exogène et dans lequel le mélange comprend en outre une sonde spécifique pour la séquence nucléotidique de ladite cible exogène.
